# EUROPEAN PATENT APPLICATION

(11) **EP 2 404 925 A1**
(43) Date of publication of application: **11.01.2012**
(21) Application number: 10305768.3
(22) Date of filing: 09.07.2010
(51) Int. Cl.: C07F 15/00, A61P 35/00

(54) **New all trans platinum (IV) complexes, their preparation and their use as antitumor agents**

(71) Applicant: Universidad Autónoma de Madrid, 28049 Madrid (ES)
(72) Inventor: Navarro Ranninger, Carmen, 28049 MADRID (ES); Alvarez-Valdes, Amparo, 28049 MADRID (ES); Quiroga, Adoracion Gomez, 28049 MADRID (ES); Vadillo, Ana, 28049 MADRID (ES); Lara, José, 28049 MADRID (ES); Mailliet, Patrick, 75013 Paris (FR); Bourrie, Bernard, 75013 Paris (FR)
(74) Representative: ABG Patentes, S.L.

(57) **Abstract**

The present invention relates to derivatives of formula (I) 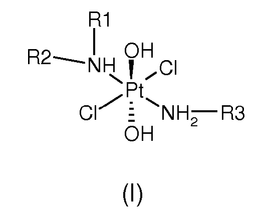 in which R1 and R2, identical or different, represent a hydrogen atom or a linear or branched C1-4 alkyl radical; provided that one of R1 and R2 is different from a hydrogen atom; R3 represents a bicyclic group of general formula (II): 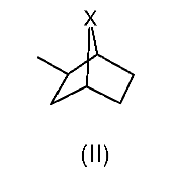 X represents O or -CH₂- or -(CH₂)₂-; in the form of a free base, of an hydrate or of a solvate.

## Description

The current invention refers to a series of new all *trans*-diamino-dichloro-dihydroxy-platinum derivatives, their preparation and their application as anticancer agents.

A few *cis*-diamino platinum complexes are widely used in cancer therapy. Antitumor properties of the first one, cisplatin, were discovered as early as 1965. Cisplatin was approved by FDA in 1978. Carboplatin and oxaliplatin were further approved by FDA in 1989 and 2002 respectively. Satraplatin and picoplatin are currently being reviewed for launching by FDA in some specific tumor settings. In cisplatin, carboplatin, oxaliplatin and picoplatin, the platinum atom, usually in the oxydation state II, is coordinated, with a *cis* geometry, to two amino ligands and to labile ligands which are either two chlorine atoms or one dicarboxylic organic acid, such as 1,1-cyclobutanedicarboxylic acid (named "CBDCA") in the case of carboplatin, or oxalic acid in the case of oxaliplatin. In the case of picoplatin, the two nitrogen ligands are α-picoline and ammonia. Satraplatin is a platinum (IV) derivative *cis*-coordinated to two amino ligands and two chlorine atom and linked to two trans-diaxial acetates.

These compounds are represented by the following formulae:

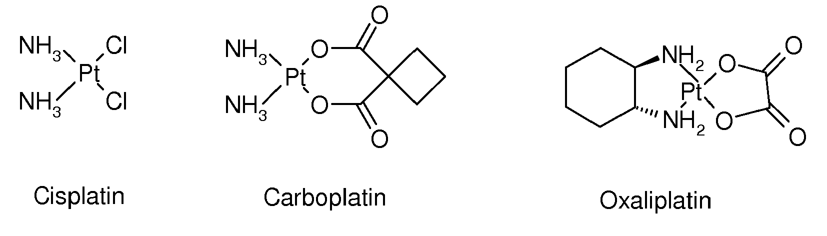

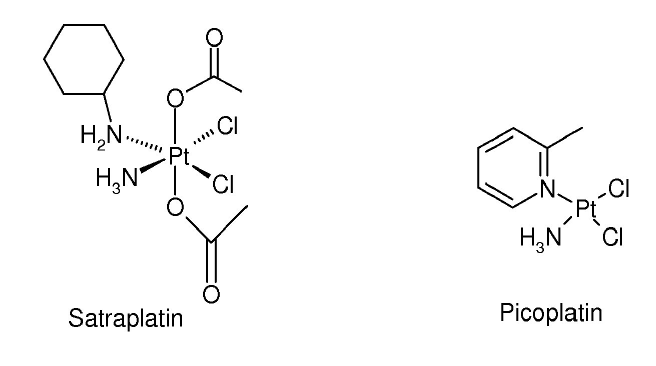

Kalinowska-Lis et al., Coordination Chemistry Reviews (2008), 252(12-14), 1328-1345 gives a general review on *trans* geometry in Platinum anti-tumor compounds.

Gonzalez-Vadillo and al., Journal of Inorganic Biochemistry (2007), 101 (4), 551-558 allows a more specific review on *trans* (II) and (IV) platinum compounds.

For many years, cis geometry in bifunctional platinum complexes has been thought to be essential for the development of antitumor platinum-based drugs, since covalent binding to 7-nitrogen atoms of two adjacent guanines on the same strand of DNA was considered as responsible for antitumor activities. The discovery of the cytostatic activity of several analogs of Transplatin (*trans* isomer of Cisplatin) implied a very important change in the structure-pharmacological activity relationships for platinum compounds, as discussed by the groups of N. Farell (Metal Ions in Biological Systems, M. Dekker, 1996, 603-639), G. Natile (Metal Ions in Biological Systems, M. Dekker, 2004, 209-250) and C. Navarro-Ranninger (Crit. Rev. Oncol. Hematol., 2000, 35, 109-120). C. Navarro-Ranninger and coworkers reported that all-*trans*-platinum (IV) complexes induce apoptosis in tumor cells resistant to Cisplatin (J. Med Chem 2007, 50, 2194-2199) and have slowed protein-binding kinetics and reactivities (Mol. Pharmacol, 2003, 63, 933-944).

ES 2214138 discloses the preparation and the antitumor activity of the all *trans* platinum (IV) complexes such as :

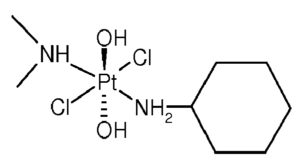

EP 0503830 A1 discloses the preparation and the antitumor activity of the all *trans* platinum (IV) complexes such as

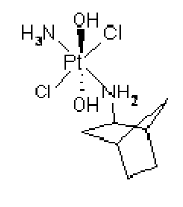

Unexpectedly, it has been found that the all-trans-diamino-dichloro-dihydroxy-platinum (IV) complexes of the invention have significantly improved antitumor activities. Thus the invention relates to new all-trans Platinum (IV) complexes of above general formula (I): 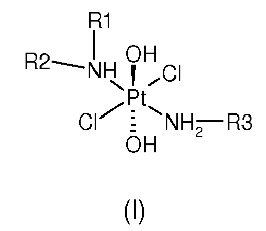 where:
- R1 and R2, identical or different, represent a hydrogen atom or a linear or branched C1-4 alkyl radical; provided that one of R1 and R2 is different from a hydrogen atom;
- R3 represents a bicyclic group of general formula (II): 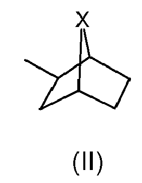
- X represents O or -CH₂- or -(CH₂)₂-;
in the form of a free base, of an hydrate or of a solvate.

The compounds of formula (I) exist in the form of pure enantiomers or racemates.

The compounds of formula (I) may also exist in the form of hydrates or solvates, namely in the form of associations or combinations with one or more water molecules or with a solvent. Such hydrates and solvates also form part of the invention.

In the context of the present invention an alkyl group is understood to mean a saturated linear or branched C1-C4 aliphatic group. By way of examples, there may be mentioned the methyl, ethyl, propyl, isopropyl, butyl, isobutyl, *tert-*butyl groups.

A first sub-group of compounds is represented by the compounds of formula (I) where:
R1 is H and R2 represents a linear or branched C1-4 alkyl radical; or R1 and R2 both are methyl.

A second sub-group of compounds is represented by the compounds of formula (I) where R1 is H and R2 is an isopropyl group.

A third sub-group of compounds is represented by the compounds of formula (I) where NH₂-R3 is the *exo-* or the *endo*-norbornylamine;
the compound of formula (I) being either in the form of a racemate or of its pure levogyre or dextrogyre enantiomer.

A fourth sub-group of compounds is represented by the compounds of formula (I) where
- R1 is H and R2 is isopropyl,
- NH₂-R3 is the exo- or the endo-norbornylamine,
   the compound of formula (I) being either in the form of a racemate or of its pure levogyre or dextrogyre enantiomer.

A fifth sub-group of compounds is represented by the compounds of formula (I) where
- R1 and R2 are methyl,
- NH₂-R3 is the *exo*-norbornylamine,
the compound of formula (I) being either in the form of a racemate or of its pure levogyre or dextrogyre enantiomer.

Among the compounds of formula (I) which are the subject of the invention, the following compounds may be mentioned in particular:
- *trans-trans-trans*-{PtCl₂(OH)₂(isopropylamine)[(*rac*)-(*exo*)-norbornylamine]} ;
- *trans-trans-trans-*{PtCl₂(OH)₂(isopropylamine)[(*rac*)-(*endo*)-norbornylamine]} ;
- *trans-trans-trans*-{PtCl₂(OH)₂(dimethylamine)[(exo)-2- norbornylamine]).

PT(IV) compounds of this invention were prepared following the next two step synthetic sequences illustrated in scheme 1:
In a first step, a compound of formula (III), where R1 and R2 are as defined above in formula (I), reacts with an amine of formula NH₂R3, where R3 is as defined above in formula (I), followed by a treatment with water and hydrochloric acid to give compound of formula (IV).
In a second step, the compound of formula (IV) reacts with hydrogen peroxide in the darkness, to give compound of formula (I). It is for example convenient for the invention to perform the reaction by heating in an inactinic glassware.

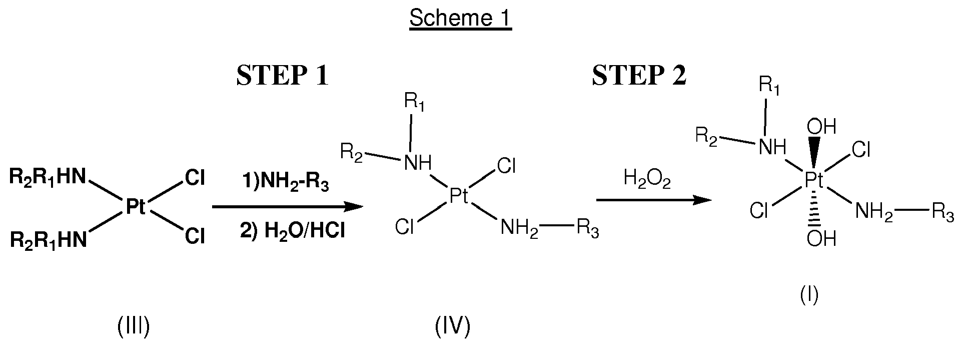

In scheme 1, the starting compounds and the reagents, when their mode of preparation is not described, are commercially available or are described in the literature, or they may be prepared according to methods described therein or which are known to persons skilled in the art.

One of the other aspects of the invention is also the compounds of formula (IV). These compounds are useful as intermediates in the synthesis of the compounds of formula (I).

The following examples describe the preparation of some compounds in accordance with the invention. These examples are not limiting and merely illustrate the present invention.

### Example 1: Synthesis of complex I with R¹=H, R²=iPr and NH₂-R³= (raC)-(exo)-norbonylamine

### Step 1: Synthesis of trans-{PtCl₂(isopropylamine)[(rac)-(exo)-2-norbornylamine)]}):

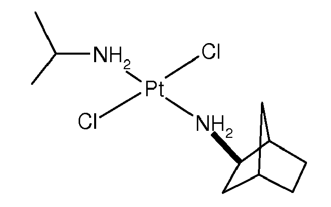

A suspension of *cis*-[PtCl₂(isopropylamine)₂] (383mg, 1.04 mmol) in water (15 mL) was treated with (rac)-(exo)-norbonylamine (0.494 mL, 4.16 mmol). The mixture was stirred at 80°C until most of the solid was dissolved and the slurry solution was maintained at the same temperature for 18 hours. The final solution obtained was filtered, washed with water and dried under reduced pressure. The solid obtained was dissolved in water (20 mL), treated with hydrochloric acid (10 mL) and heated at 90 °C for 24h. After cooling down to room temperature, the product was collected by filtration as a yellow solid and washed with water (4x10 mL), obtaining 320 mg of pure compound, previous drying the yellow solid at 65°C under reduce pressure for 12 hours (5 mmHg).
*trans*-{PtCl₂(isopropylamine)[(*rac*)-(*exo*)-2-norbornylamine)]}. Yield: 70%.
Elemental analysis for C₁₀H₂₂N₂Cl₂Pt·3/2H₂O
Calculated C: 25.92, H: 5.44, N: 6.05
Found C: 25.82, H: 4.86, N: 6.30
¹H NMR (CDCl₃)(ppm): 3.60-3.23 (m, 3H), 3.05 (m, 1H), 2.46 (s, 1H), 2.31 (s, 1H), 1.80 (ddd, J = 13.2, 7.8, 2.0 Hz, 1H), 1.51-1.31 (m, 4H), 1.33 (d, J = 6.2 Hz, 6H), 1.28-1.15 (m, 2H), 1.15-1.10 (m, 1H).

### Step 2: Synthesis of trans-trans-trans-{PtCl₂(OH)₂(isopropylamine)[(rac)-(exo)-norbornylamine])

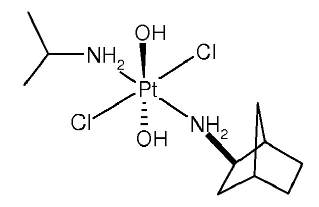

### Example 1

The complex *trans*-{PtCl₂(isopropylamine)[(*rac*)-(*exo*)-2-norbornylamine]} (300mg, 0.685 mmol), prepared at step 1, was suspended in water (20 ml) and hydrogen peroxide (35%, 1.0 ml) was added. The mixture was stirred and heated in the darkness at 70°C. After cooling, the product was collected by filtration as a pale yellow solid, washed with cold water and chloroform, and dried under reduced pressure.

*Trans-trans-trans-*{PtCl₂(OH)₂(isopropylamine)[(*rac*)-(*exo*)-norbornylamine]} Yield: 40%.
Elemental analysis for C₁₀H₂₄N₂Cl₂O₂Pt·H₂O·1/2KCl
Calculated C: 22.85, H: 4.99, N: 5.33
Found C: 22.48, H: 4.60, N: 5.43
IR (cm⁻¹): 3428 (broad signal), 2959, 1636, 1034, 798.

### Example 2: Synthesis of complexes I with R¹=H, R²=iPr and NH₂-R³= (rac)-(endo)-norbonylamine

### Step 1: Synthesis of trans-{PtCl₂(isopropylamine)[(rac)-(endo)-2-norbornylamine]}

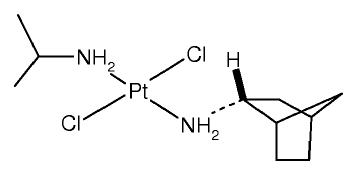

To a solution of (rac)-(endo)-norbonylamine hydrochloride (767mg, 5.20 mmol) and KOH (583 mg, 10.4 mmol) in H₂O (50 mL) (previously cooled to 0°C for 15 min) was added a solution of *cis*-[PtCl₂(isopropylamine)₂] (500 mg, 1.30 mmol) in water (5 mL). The mixture was stirred at 80°C until most of the solid was dissolved and the blur solution was maintained at the same temperature for 18 hours. The final solution was filtered, washed with water and dried under reduced pressure. The solid obtained was dissolved in water (20mL), treated with hydrochloric acid (10 mL) and heated at 90°C for 24h. After cooling down to room temperature, the product was collected by filtration and washed with water (4 x 10 mL), obtaining a yellow solid, previous drying at 65 °C under reduced pressure for 12 hours (5 mmHg).
*trans*-{PtCl₂(isopropylamine)[(*rac*)-(*endo*)-2-norbornylamine]} Yield: 65% Elemental analysis for C₁₀H₂₁N₂Cl₂Pt·1/3KCl-2/3H₂0
Calculated C: 25.39, H: 4.97, N: 5.92
Found C: 25.35, H: 4.69, N: 6.24
¹H NMR (CDCl₃)(ppm): 3.84-3.41 (bs, 3H), 3.36-3.22 (m, 1H), 2.59 (s, 1H), 2.21 (s, 1 H), 2.13-2.05 (m, 1H), 1,67-1.42 (m, 6H), 1.33 (d, *J* ₌ 6.4 Hz, 6H), 1.24 (t, J= 9.1 Hz, 1H), 1.01 (dt, J= 13.0, 3.1 Hz, 1H).

### Step 2: Synthesis of trans-trans-trans-{PtCl₂(OH)₂(isopropylamine)[(rac)-(endo)-norbornylamine]}

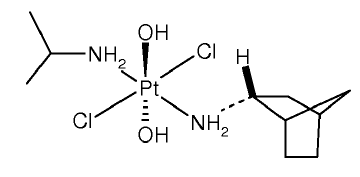

### Example 2

A solution of *trans*-{PtCl₂(isopropyl)[(*rac*)-(*endo*)-norbornylamine]} (80mg, 0.10 mmol), prepared at step 1, was suspended in water (10 ml) and hydrogen peroxide (2 mL, 18.3 mmol) was added. The mixture was stirred and heated, in the darkness, at 90 ºC for 24 h. The residue solution was concentrated, acetone was added and the solution was left in the fridge for 12 hours. The obtained solid was washed with acetone and dried under reduced pressure.
*Trans-trans-trans*-{PtCl₂(OH)₂(isopropylamine)[(*rac*)-(*endo*)-norbornylamine]} Yield: 19%
Elemental analysis for C₁₀H₂₄N₂O₂Cl₂Pt·.2H₂O
Calculated C: 23.72, H: 5.57, N: 5.53
Found C: 23.92, H: 4.84, N: 5.90
IR (cm⁻¹): 3435 (bs), 2860, 1636, 616

### Example 3: Synthesis of complex I with R¹= R²= methyl and NH₂-R³= (exo)-2-norbonylamine

### Step 1: Synthesis of trans-{PtCl₂(dimethylamine)[(exo)-2-norbornylamine]}:

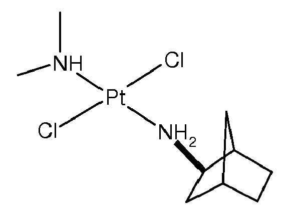

A suspension of *cis*-[PtCl₂(dimethylamine)_{2]} (Journal of the Chemical Society, Chemical Communications 1987 (6), 443-445) (425 mg; 1.2 mmol) in water (25 mL) and (*rac*)-(*exo*)-2-norbornylamine (634 µL; 5.3mmol) was stirred at 90 ºC. A white precipitation was observed at 16 hours, and after 20h, the reaction was cooled down to room temperature and filtered. The milky solution was removed and the liquid was evaporated under reduced pressure. The white crystalline residue was suspended in water, treated with HCl (1.3mL; 4.5 mmol) and heated at 100 ºC for 72 hours. Then the reaction was cooled down to room temperature and the procedure explained before was repeated (HCl (1.3mL; 4.5mmol) addition and heated for 24h). After cooling at room temperature, the bright yellow solid was filtered and washed with cold water and acetone. The solid was then dissolved in chloroform and precipitated with hexane. The final solid was dried under vacuum.
*trans*-{PtCl₂(dimethylamine)[(exo)-2-norbornylamine]} Yield: 35%
Elemental analysis for C₉H₂₀N₂Cl₂Pt·.2,5% CH₃COCH₃
Calculated C: 25.77, H: 4.79, N: 6.60
Found C: 26,28, H: 4,75, N: 6,33
¹H NMR (CDCl₃)(ppm): 3.84-3.41 (bs, 1H), 3.25-3.17 (m, 2H), 3.06 (s, 1H), 2.66 (s, 3H), 2,64 (s, 3H) 2.45 (s, 1H), 2.29 (s, 1H), 1.83 (ddd, J = 13.7, 7.6, 1.9 Hz, 1H), 1.58-1.42 (m, 3H), 1.37-1.24 (m, 1H), 1.21-1.10 ( m, 2H), 1,12-1,06 (m, 1H).
¹⁹⁵Pt NMR (CDCl₃)(ppm): -2187,93

### Step 2: Synthesis of trans-trans-trans-{PtCl₂(OH)₂(dimethylamine)[(exo)-2-norbornylamine])

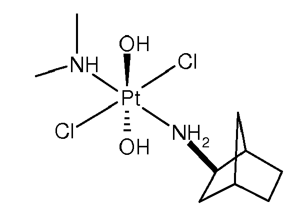

### Example 3

A suspension of *trans*-[PtCl₂(dimethylamine)(*exo*)-2-norbornylamine] (158 mg; 0.37 mmol), prepared at step 1, in water (12 mL) and hydrogen peroxide solution (1.0 mL; 9 mmol) was stirred at 90 ºC for 4 hours and overnight at room temperature in the dark. The yellow solid resulted from the reaction, was filtered and washed with water and finally dried under reduced pressure.
*Trans-trans-trans*-{PtCl₂(OH)₂(dimethylamine)[(exo)-2- norbornylamine]} Yield: 18%
Elemental analysis for C₉H₂₂N₂Cl₂O₂Pt
Calculated C: 23.72, H: 4.87, N: 6.13
Found C: 22.66, H: 4.61, N: 5.85
¹H NMR (CDCl₃)(ppm): 3.02 (bs, 1H), 2.42-2.50 (m, 7H), 2.27 (bs, 1H), 1.75-1.72 (m, 1H), 1.54-1.48 (m, 4H), 1,22-1.14 (m, 3H).

The *in vitro* and *in vivo* antitumor activities of compounds from the invention have been evaluated against a panel of cisplatin-sensitive and cisplatin-resistant cell lines and/or tumors, according to the following assay conditions:

### Cell lines and tumours:

- CCRF-CEM is a human T-cell leukemia cell line (DSMZ ref ACC 240), established in 1964 from peripheral blood of 3-years old young girl bearing a terminal-stage ALL *(*Foley et al., Cancer 18: 522-529 (1965)).
- NCI-H460 is a human epidermoïd lung tumour cell line, obtained from the NCI (NCI-Frederick. Tumor Bank).
- MX1 is a human mammary carcinoma tumour, harbouring BRCA1 mutation, obtained from NCI (NCI-Frederick. Tumor Bank).

### In vitro antiproliferative assays:

Tumour cells were grown in appropriate culture medium, either RPMI-1640 or DMEM, complemented with 10% calf-fetal serum and antibiotics.

Cell proliferation was evaluated by ¹⁴C-thymidine uptake in DNA. Exponentially growing cells - from 5.10³ to 10⁴ per well in 180 µL culture medium - were introduced in a 96-wells microtitration Cytostar-T apparatus (GE Healthcare Bio-Sciences, Uppsala, Sweden). After 4 hours pre-incubation under CO₂ atmosphere at 37°C, compounds were added in 10 µL volume. After incubation for an additional 72 hours period, 0.1 µCi ¹⁴C-thymidine was added in each well, C¹⁴ -thymidine uptake was recorded 24 hours later on a scintillation counter (Perkin-Elmer Life Sciences, Boston, MA, USA). IC₅₀, defined as the dose which inhibits 50% of cell proliferation, were thus determined.

Table 1 summarizes the *in vitro* antiproliferative data obtained with compounds from the invention, in comparison with various reference compounds - cisplatin, oxaliplatin and satraplatin - and a compound representative from ES 2214138.

**Table 1**

| Compound | CEM IC50 µM | H460 IC50 µM |
|---|---|---|
| 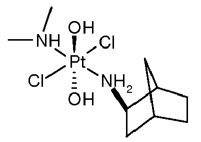 | 0.031 | 0.315 |
| EXAMPLE 3 | | |
| 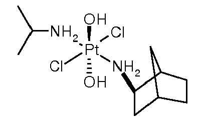 | 0.053 | 0.532 |
| EXAMPLE 1 | | |
| 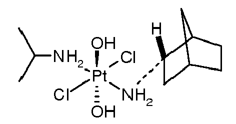 | 0.025 | 0.255 |
| EXAMPLE 2 | | |
| cisplatin | 0.4-0.8 | 2.6-4 |
| oxaliplatin | 4 | 2.4 |
| satraplatin | 0.4 | 0.19 |
| 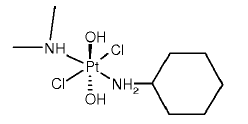 | n.d. | n.d. |

| | | |
|---|---|---|
| *n.d means not determined.* | | |

### In vivo antitumor evaluation of Example 1 in MX1 tumor model.

7 to 8 weeks-old female SCID mice, obtained from Charles River (L'Arbresle, Lyon , France) were acclimated for at least 1 week previous experiment.

Animals, placed under normal light cycle, had then *ad libitum* access to food and water. MX1 cells, 10⁷ cells per mouse initially grown in RPMI medium supplemented with 10% foetal-calf serum and antibiotics, were subcutaneously implanted in mice. When 1000 mg tumour volume was reached, fragments (2-3 mm diameter) were cut, placed in phosphate buffer (PBS), bilaterally implanted and further passaged in mice until tumour growth rate is stable, before starting the experiment. Tumors were measured with a caliper twice weekly until the tumor reached 2000 mm³ or until the animal died (which ever come first). Tumor volumes were estimated from 2 dimensional measurements: Volume (in mm³) = (a x b²)/2, where a and b are the tumor length and width (mm) respectively. The variation from the baseline of the tumoral volume is calculated in the treated and control group. These values are used to calculate the median in the treated group (ΔT) and in the control group (ΔC). ΔT/ΔC, expressed as a percentage, is the ratio of median at a chosen day (the last day before it becomes necessary to sacrifice control mice owing to tumor size). ΔT/ΔC values can be translated into an activity rating, according to the Southern Research Institute (SRI) criteria:

| **SRI activity criteria** | **ΔT/ΔC** |
|---|---|
| Highly active - % regression is dated | <0 |
| | |
| Very active | <10% |
| | |
| Active | 10%< <40% |
| | |
| inactive | >40% |

When ΔT/ΔC values are negative, the percentage of regression is evaluated. A Partial regression (PR) is defined as a decrease in tumor volume greater than or equal to 50% of the tumor volume at start of treatment. A Complete regression (CR) is defined as a decrease in tumor volume below the limit of palpation (T=10mm³). At the end of the study, the number of tumor free survivor (TFS) which correspond to mice without any detectable tumor is determined. Both drug-related deaths and maximum percent relative mean net body weight loss are also determined. A body weight loss (mean of group) of greater than 15% for more than 3 consecutive days or 20% weight loss for one day or 10% drug deaths are considered to indicate an excessively toxic dosage.

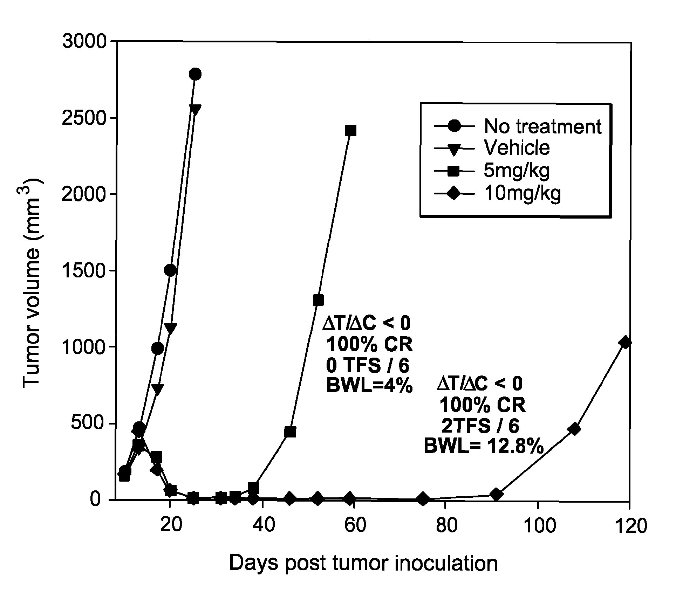

As depicted in Figure 1 (which shows the effect of example 1 compound, administered by IV route, in Scid mice bearing the MX1 tumor), compound from example 1 is highly active on MX1, a human mammary tumour xenografted in SCID mice. Compound of example 1 administered by i.v. route daily during 5 days at 5 or 10 mg/kg (25 or 50 mg/kg total dose) from day 10 to day 15 post tumor implantation was highly active : a ΔT/ΔC<0 was observed at both dosages and 100% CR was observed. At 10 mg/kg, 2 TFS were noted at the end of the study. At these dosages, 4% and 12% body weight loss with no drug death was observed, indicating no significant toxicity. Administered at 20 mg/kg, compound of example 1 was detected to be toxic for mice, with 6/6 deaths detected respectively on days 17, 19, 20, 21 and 24 (2 mice). This suggests that 10 mg/kg can be considered as the maximal tolerated dose in this schedule of administration (daily for 5 days).

MX1 xenograft model is sensitive to platinums, and part of this sensitivity could be attributed to the dysfunction of DNA repair enzymes (Donawho CK et al Clin Cancer Res 2007; 13:2728-2737). MX1 has BRCA1 deletions and contains a BRCA2 mutation. These genes are important for DNA double-strand break repairs (Caldecott KW et al Nat Struct Mol Biol 2005; 12:387-388.) and these genetic alterations lead to a predisposition to breast and ovarian cancer (Weberpals et al J. Clin Oncol 2008; 19: 3259-3267; Futreal PA et al Science 1994;266:120-122; Freidman LS et al Nat Genet 1994; 8:399-404; Fedier A et al, Int J Oncol 2003; 22:1169-1173; Couch FJ et al N. Engl J Med 1997; 336:1409-1415; Yang Q et al Cancer 2001; 92:54-60; Hilton JL et al J Natl Cancer Inst 2002; 94:1396-1406). Thus, there is a rational to use the new platinum derivatives of the invention in BRCA-associated tumors, mainly breast and ovarian tumors. The new platinum derivatives of the invention may also be used in combination with PARP inhibitors in those pathologies.

The compounds according to the invention may therefore be used as medicaments or for the preparation of medicaments, in particular for the treatment of BRCA-associated tumors, such as breast and ovarian tumors.

Thus, according to another of its aspects, the subject of the invention is medicaments which comprise a compound of formula (I), or a hydrate or a solvate of the compound of formula (I).

These medicaments find use in therapy, in particular in the treatment of cancers. They are particularly active in the treatment of solid cancers such as breast and ovarian cancers.

According to another of its aspects, the present invention relates to pharmaceutical compositions comprising, as active ingredient, a compound according to the invention. These pharmaceutical compositions contain an effective dose of at least one compound according to the invention, a hydrate or a solvate of the said compound, and at least one pharmaceutically acceptable excipient.

The said excipients are chosen, according to the pharmaceutical dosage form and the desired mode of administration, from the customary excipients which are known to persons skilled in the art.

In the pharmaceutical compositions of the present invention for subcutaneous, intramuscular or intravenous administration, the active ingredient of formula (I) above, or its possible solvate or hydrate, may be administered in unit dosage form, mixed with conventional pharmaceutical excipients, to animals and to human beings for the treatment of cancer diseases or disorders.

The appropriate unit dosage forms comprise the forms for parenteral, in particular subcutaneous, intramuscular or intravenous administration.

By way of example, a unit dosage form for a compound according to the invention in the form of a solution may comprise the following components:

| | |
|---|---|
| Compound according to the invention | 10 to 100 mg |
| Water for injection | 2 to 20 ml |

According to another embodiment of the invention, the compound may be provided in the form of a lyophilisate.

It is preferable to use the compounds according to the invention intravenously at doses of between 10 and 200 mg/m². There may be special cases where higher or lower dosages are appropriate; such dosages do not depart from the scope of the invention. According to the usual practice, the dosage appropriate for each patient is determined by the doctor according to the mode of administration, the weight and the response of the said patient.

The present invention, according to another of its aspects, also relates to a method for treating the pathologies indicated above, which comprises the administration, to a patient, of an effective dose of a compound according to the invention, in the form of a free base, of an hydrate or of a solvate.

## Claims

1. A compound of general formula (I) : 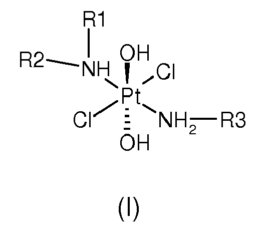 in which:
R1 and R2, identical or different, represent a hydrogen atom or a linear or branched C1-4 alkyl radical; provided that one of R1 and R2 is different from a hydrogen atom;
R3 represents a bicyclic group of general formula (II): 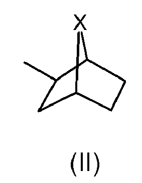
X represents O or -CH₂- or -(CH₂)₂-;
in the form of a free base, of an hydrate or of a solvate.

2. A compound of formula (I) according to Claim 1, **characterized in that** R1 is H and R2 represents a linear or branched C1-4 alkyl radical; or R1 and R2 both are methyl.

3. A compound of formula (I) according to Claim 1 or 2, **characterized in that** R1 is H and R2 is an isopropyl group.

4. A compound of formula (I) according to Claim 1, **characterized in that** NH₂-R3 is the *exo-* or the *endo*-norbornylamine; the compound of formula (I) being either in the form of a racemate or of its pure levogyre or dextrogyre enantiomer.

5. A compound according to Claim 1, where
- R1 is H and R2 is isopropyl;
- NH₂-R3 is the *exo-* or the *endo*-norbornylamine;
the compound of formula (I) being either in the form of a racemate or of its pure levogyre or dextrogyre enantiomer.

6. A compound according to Claim 1, where
- R1 and R2 are methyl;
- NH₂-R3 is the *exo*-norbornylamine;
the compound of formula (I) being either in the form of a racemate or of its pure levogyre or dextrogyre enantiomer.

7. A compound according to Claim 1, chosen among:
- *trans-trans-trans*-{PtCl₂(OH)₂(isopropylamine)[(*rac*)-(*exo*)-norbornylamine]};
- *trans-trans-trans*-{PtCl₂(OH)₂(isopropylamine)[(*rac*)-(*endo*)-norbornylamine]}; and
- *trans-trans-trans*-{PtCl₂(OH)₂(dimethylamine)[(exo)-2- norbornylamine]).

8. Process for the preparation of a compound of formula (I) as defined in claim 1, where the compound of formula (IV) 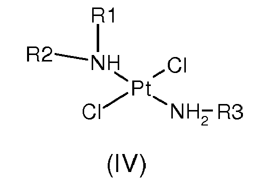 where R1, R2 and R3 are as defined in formula (I) according to claim 1, reacts with hydrogen peroxide in the darkness to give compound of formula (1).

9. Process for the preparation of a compound of formula (IV) as defined in claim 8, where the compound of formula (III) 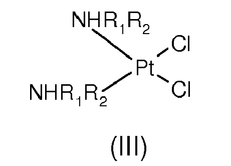 where R1 and R2 are as defined in formula (I) according to claim 1,
reacts with an amine of formula NH₂R3, where R3 is as defined in formula (I) according to claim 1,
followed by a treatment with water and hydrochloric acid to give compound of formula (IV).

10. Compound of formula (IV) 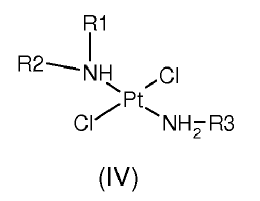 in which:
R1 and R2, identical or different, represent a hydrogen atom or a linear or branched C1-4 alkyl radical
R3 represents a bicyclic group of general formula (II): 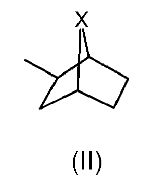
X represents O or -CH₂- or -(CH₂)₂-

11. Medicament, **characterized in that** it comprises a compound of formula (I) according to any one of Claims 1 to 7, a hydrate or a solvate of the compound of formula (I).

12. Pharmaceutical composition, **characterized in that** it comprises a compound of formula (I) according to any one of Claims 1 to 7, a hydrate or a solvate of this compound, and at least one pharmaceutically acceptable excipient.

13. A compound of formula (I) according to any one of Claims 1 to 7, for its use for the treatment of cancer.
